# EUROPÄISCHE PATENTSCHRIFT

(11) **EP 2 561 769 B1**
(45) Veröffentlichungstag und Bekanntmachung des Hinweises auf die Patenterteilung: **01.11.2017**
(21) Anmeldenummer: 12180509.7
(22) Anmeldetag: 23.09.2008
(51) Int. Cl.: A23L 3/00, A23L 3/10, A23L 3/14, A61L 2/04, A61L 2/07, D21H 17/00, D21H 19/10, D21H 21/16, D21H 27/00, D21H 27/30, B32B 27/10, B65D 5/00, B65D 65/38

(54) **Quaderförmige Karton/Kunststoff-Verbundpackung zur Verwendung in einer Vorrichtung zum Sterilisieren von quaderförmigen Karton/Kunststoff-Verbundpackungen in einem Autoklaven**
Emballage composite carton/plastique carré destiné à être utilisé dans un dispositif de stérilisation d'emballages composites carton/plastique carrés dans un autoclave
Square-shaped cardboard/plastic compound packaging for use in a device for sterilising square-shaped cardboard/plastic compound packages in an autoclave

(30) Priorität: 24.09.2007 DE 102007045720
(43) Veröffentlichungstag der Anmeldung: 27.02.2013
(62) Teilanmeldung aus: 08804615.6
(73) Patentinhaber: SIG Technology AG, 8212 Neuhausen am Rheinfall (CH)
(72) Erfinder: Wolters, Michael, 52525 Heinsberg (DE); Dintelmann, Thomas, 64380 Rossdorf (DE)
(74) Vertreter: Cohausz & Florack

(56) Entgegenhaltungen:
- EP-A- 0 724 887
- EP-A- 1 382 353
- EP-A- 1 862 082
- WO-A-02/28721
- WO-A-98/16431
- WO-A-02/090206
- WO-A-03/103417
- FR-A- 2 290 249
- GB-A- 759 238
- GB-A- 762 335
- GB-A- 2 040 668
- JP-A- 9 028 772
- JP-A- 9 215 733
- JP-A- 10 033 643
- JP-A- 2001 231 520
- JP-A- 2002 101 862
- JP-A- 2003 319 768
- US-A- 5 705 127

## Beschreibung

Die Erfindung betrifft eine quaderförmige Karton/Kunststoff-Verbundpackung zur Verwendung in einer Vorrichtung zum Sterilisieren von quaderförmigen Karton/Kunststoff-Verbundpackungen in einem Autoklaven, wobei die aus Produkt und Verpackung bestehenden Packungen einer Wärmebehandlung bei einer bestimmten Temperatur über eine bestimmte Dauer unterzogen wird, wobei das Produkt aus einer Flüssigkeit und stückigen Anteilen besteht und wobei im Inneren des Autoklaven Einrichtungen zum rotatorischen Antrieb der Packungen um eine Drehachse vorgesehen sind, wobei eine Mehrzahl von in parallelen Reihen dicht benachbarter Packungen (P) auf Tragböden angeordnet sind und wobei eine Mehrzahl von mit Packungen (P) bestückten Tragböden übereinander angeordnet und in ihrer gegenseitigen Lage fixiert sind.

Es ist seit langem bekannt, portioniert abgepackte Produkte wie beispielsweise Lebensmittel zur Haltbarmachung einem so genannten Autoklavierprozess zu unterziehen. Hierbei werden beispielsweise Konservendosen, in welche zuvor Lebensmittelprodukte abgepackt worden sind in einem Autoklaven für eine bestimmte Dauer einer bestimmten Temperatur ausgesetzt, um die im Produkt bzw. der Dose befindlichen Keime zuverlässig abzutöten. Konservendosen haben sich für den genannten Zweck insbesondere deshalb bewehrt, weil sie unempfindlich gegenüber den in einem Autoklaven herrschenden Bedingungen (hohe Temperatur, hohe Feuchte, hoher Druck) sind.

Werden Lebensmittel oder andere zu sterilisierende Produkte jedoch nicht in Konserven sondern in anderen Verpackungen auf den Markt gebracht so muss - mit relativ hohem Aufwand - zunächst die Verpackung sterilisiert werden, dann das Produkt unter sterilen Bedingungen in die Verpackung gefüllt werden und schließlich die Verpackung aseptisch geschlossen werden. Auf diese Weise hergestellte Karton/Kunststoff-Verbundpackungen sind in der Lage, Produkte wie beispielsweise Milch oder püriertes Gemüse über einen längeren Zeitraum aufzunehmen.

Aus der EP 1 382 353 ist eine Vorrichtung zum Sterilisieren von Karton/Kunststoff-Verbundpackungen bekannt. Dabei werden mit Hilfe eines Rotationsautoklavprozesses zum Sterilisieren von Lebensmitteln die Verbundpackungen in einem Autoklaven währen der Behandlung derart bewegt, dass eine ständige Durchmischung des Packungsinhaltes während des Autoklavierprozesses gewährleistet ist. Hierbei unterliegen die Packungen einer erhöhten Beanspruchung durch Feuchtigkeit, Druck und Temperatur, insbesondere an ihren Nähten und Schnittkanten.

Des Weiteren ist ein sterilisierbares Verbundmaterial aus der WO 02/090206 A1 bekannt. Dort werden die einzelnen Schritte zum Herstellen einer Karton/Kunststoff-Verbundpackung aufgeführt. Eine quaderförmige Packungsform wird dort nicht offenbart. Darüber hinaus enthält WO 02/090206 A1 nichts, was auf eine rotierende Behandlung beim Autoklavieren hindeuten könnte.

Davon ausgehend liegt der vorliegenden Erfindung die Aufgabe zu Grunde, die eingangs genannten Packungen so auszugestalten und weiterzubilden, dass eine zufrieden stellende Sterilisation einer größeren Anzahl von Packungen ermöglicht wird, ohne dass die Packungen verformt werden, dass ihr Druckbild beschädigt wird oder dass Wasser bzw. Dampf in sie eindringt.

Diese Aufgabe ist dadurch gelöst, dass der Karton zur Verminderung des Wassereintritts mittels Leim hydrophobiert ist und dass die eingesetzte Leimmenge zwischen 1 und 4 kg/t trockenen Faserstoffs liegt.

Eine weitere Lehre der Erfindung sieht vor, dass die Dichte des verwendeten Rohkartons im Bereich von 680 bis 860 kg/m³ liegt. Vorzugsweise beträgt der Mahlgrad der für die Packung verwendeten Kartonfasern 15 bis 35 SR. Ein solches Material kann den im Autoklaven herrschenden extremen Bedingungen gut standhalten.

Gemäß einer weiteren Ausbildung der Erfindung sind die Oberflächen der Packungen hydrophob bedruckt und/oder ist der Druck durch eine transparente Kunststoffschicht geschützt. Eine Beschädigung des Druckbildes durch Abrieb und Abplatzen oder Verfärben ist mit dieser Ausgestaltung zuverlässig ausgeschlossen.

Eine besonders gute Sterilisation lässt sich dann erreichen, wenn die Füllmenge der verwendeten Packungen so gewählt ist, dass in ihrem Inneren eine Gasblase von 2 bis 30 % des Nennvolumens der Packung vorhanden ist. Dadurch und die im Produkt befindlichen stückigen Anteile findet beim Rotieren im Autoklaven eine gleichmäßige und ständige Durchmischung des Packungsinhaltes statt. Dies führt zu deutlich kürzeren Behandlungszeiten, was eine weitere Schonung der verwendeten Packungen nach sich zieht. Als Gas lässt sich in bekannter Weise beispielsweise Stickstoff einsetzen.

Eine weitere Ausbildung der Erfindung sieht vor, dass die Schnittkanten der Packungen zusätzlich verpresst sind. Während die Packungsoberflächen durch die PP-Beschichtung und/oder die hydrophobe Bedruckung relativ gut vor Eindringen von Feuchtigkeit geschützt sind, sind die "offenen" Schnittkanten im Bereich der Nähte besonders gefährdet. Aus diesem Grunde verhindert eine zusätzliche Verpressung das Eindringen von Dampf oder Feuchtigkeit in das Verbundmaterial.

Zum Abfangen des Sauerstoffanteils im Karton/Kunstoff-Verbundmaterial können nach einer weiteren Lehre der Erfindung Scavenger eingesetzt werden. Die Verwendung solcher "Sauerstofffänger" in Packstoffmaterial ist für sich bereits bekannt.

Die Erfindung wird nachfolgend anhand einer lediglich ein Ausführungsbeispiel darstellenden Zeichnung näher erläutert. In der Zeichnung zeigen
- Fig. 1:: eine Ausführungsform einer bevorzugten Vorrichtung zum Sterilisieren von erfindungsgemäßen Packungen in Stirnansicht in einem nur schematisch angedeuteten Autoklaven,
- Fig. 2:: die verwendete Vorrichtung aus Fig. 1 in Seitenansicht,
- Fig. 3:: die verwendete Vorrichtung aus Fig. 1 in Draufsicht, jedoch mit geöffnetem Deckel, der den Blick auf die liegend angeordneten erfindungsgemäßen Packungen freigibt,
- Fig. 4:: einen vergrößerten Querschnitt durch den oberen Teil der verwendeten Vorrichtung, in Richtung der Pfeile IV-IV in Fig. 2,
- Fig. 5:: einen Eckausschnitt eines leeren Einsatzbleches in perspektivischer Darstellung und
- Fig. 6:: den Eckausschnitt aus Fig. 5 mit einer eingesetzten erfindungsgemäßen Packung.

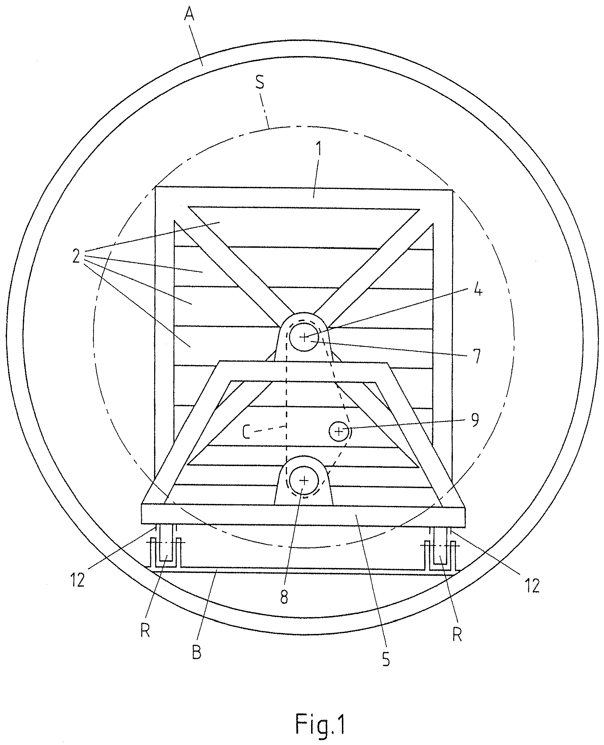

Die gezeigte Vorrichtung zum Sterilisieren von quaderförmigen Karton/Kunststoff-Verbundpackungen P besteht, wie aus den Fig. 1 bis 3 ersichtlich, zunächst aus einem Rahmengestell 1, in welches eine Mehrzahl von Einsatzblechen 2 eingesetzt sind. Damit die Einsatzbleche 2 beim Rotieren des Rahmengestells 1 nicht aus diesem herausfallen, besitzt das Rahmengestell 1 einen arretierbaren Deckel 3.

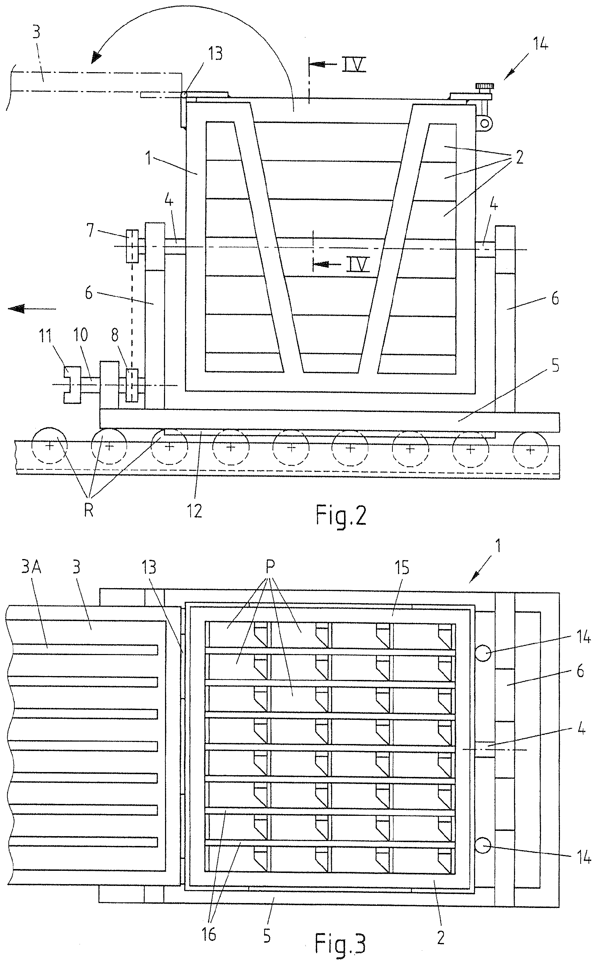

Das Rahmengestell 1 ist über Achsen 4 in auf einem Grundrahmen 5 befindlichen seitlichen Stützen 6 drehbar gelagert. Zum Aufbringen des notwendigen Drehmoments ist die in der Fig. 2 links angeordnete Drehachse 4 endseitig mit einem Kettenrad 7 versehen, welches über eine gestrichelt dargestellte Kette C mit einem weiteren Kettenrad 8 sowie einem nur in Fig. 1 angedeuteten Spannrad 9 mit einer Antriebswelle 10 verbunden ist, welche endseitig eine Klauenkupplung 11 aufweist, die beim Hineinfahren in den Autoklaven A in Richtung des nicht näher bezeichneten Pfeils mit einer entsprechenden Kupplung eines Antriebsmotors (nicht dargestellt) in Wirkverbindung tritt.

Zum Erleichtern des Beschickungsvorganges verfügt der Grundrahmen 5 an seiner Unterseite über Führungsschienen 12, die zur Führung entlang an einer Basis B des Autoklaven A freidrehend befestigten Rollen R dienen, wie dies aus den Fig. 1 und 2 deutlich hervorgeht.

Aus Fig. 2 ist ferner zu entnehmen, dass der Deckel 3 mittels eines Gelenks 13 am Rahmengestell 1 befestigt ist und mit Hilfe von zwei Verschlüssen 14 in seiner geschlossenen Stellung arretiert werden kann. Das geschlossene Rahmengestell 1 lässt sich dann im Inneren des Autoklaven A um die Rotationsachse 4 drehen; der zugehörige Schwenkkreis S ist in Fig. 1 strichpunktiert dargestellt.

Aus Fig. 3 geht nun hervor, dass jedes Einsatzblech 2 von denen nur das oberste bei aufgeklapptem Deckel 3 sichtbar ist, über einen umlaufenden Rahmen 15 verfügt, der die seitliche Begrenzung einer Vielzahl erfindungsgemäßer Packungen P bildet. Damit die Packungen P in gleichmäßigen Längsreihen hintereinander und auf ihren Schmalseiten liegend innerhalb des Einsatzbleches 2 angeordnet werden können, verfügt jedes Einsatzblech 2 über die gesamte Länge des Einsatzbleches 2 verlaufende Stegbleche 16, die eine seitliche Begrenzung für die benachbarten Packungsreihen darstellen.

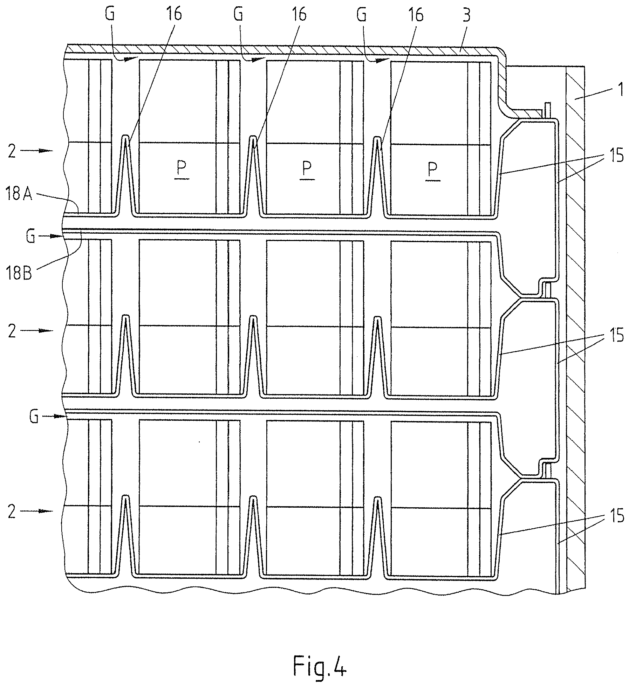

Der genaue Aufbau der Einsatzbleche 2 lässt sich aus Fig. 4 entnehmen, in der der obere Teil eines Paketes von Einsatzblechen 2 aus Fig. 2 im Querschnitt in Richtung der Pfeile IV-IV gezeigt ist. Hier ist zunächst zu erkennen, dass der Rahmen 15 der Einsatzbleche 2 hohl ausgebildet ist und so weit hinunter reicht, dass er gewissermaßen gleichzeitig einen oberen "Deckel" für die unter ihm angeordneten Packungen P darstellt. Der Deckel 3 besitzt daher an seiner Unterseite die gleiche Form. Ferner ist erkennbar, dass die Stegbleche 16 so angeordnet sind, dass sie nach oben spitz zulaufen, unten jedoch jeweils eine Packungsbreite definieren. Ein solcher Aufbau ermöglicht eine leichte seitliche Bewegung der Packungen P beim Drehvorgang im Autoklaven A.

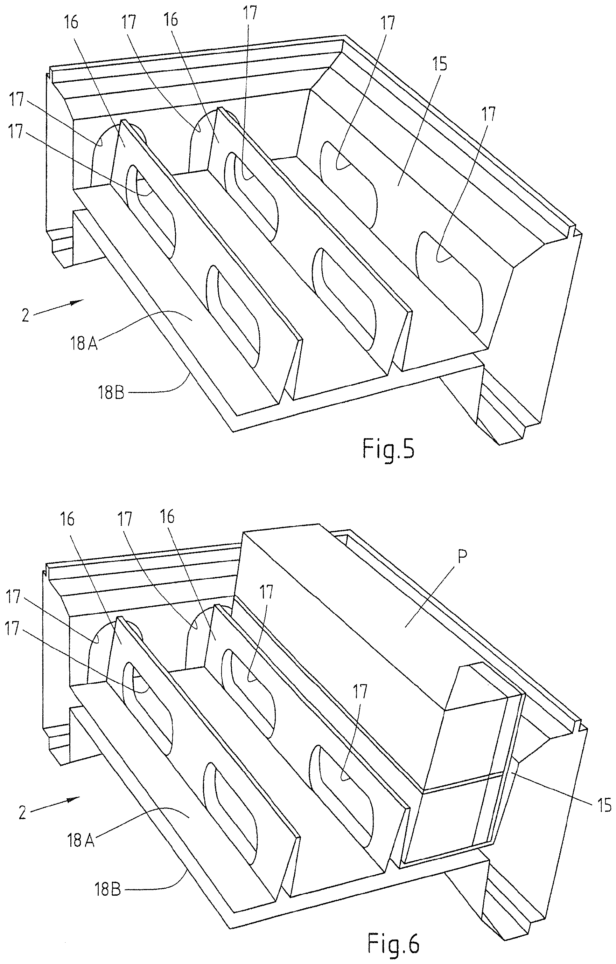

Damit sich nun das Sterilisiermedium optimal auch im Bereich der Einsatzbleche 2 verteilen kann, weisen sowohl der Rahmen 15 als auch die Stegbleche 16 Ausnehmungen 17 auf. Der Boden 18 der Einsatzbleche 2 ist als Hohlboden ausgeführt, d. h. hier besteht aus einem oberen Boden 18A und einem unteren Boden 18B wie in den perspektivischen Darstellungen gemäß Fig. 5 und Fig. 6 deutlich gezeigt. Diese Ausbildung hat den Vorteil, dass die geschlossenen Böden 18A, welche nicht mit Ausnehmungen oder Löchern versehen sind, eine optimale Abstützung der schmalen Längsseiten der erfindungsgemäßen Packungen P darstellen. Zur besseren Übersicht ist in Fig. 6 eine in das gezeigte Einsatzblech 2 eingesetzte Packung P dargestellt.

Dadurch, dass oberhalb des gezeigten Einsatzbleches 2 ein weiteres Einsatzblech 2 bzw. der Deckel 3, welcher ebenfalls mit Schlitzen 3A versehen ist, die Packungsreihen nach oben hin begrenzen, erfolgt auch eine entsprechende Abstützung bei der Rotation des Rahmengestells 1. Die Schlitze 3A sind, wie aus Fig. 3 hervorgeht, dazu genau oberhalb der Stegbleche 16 angeordnet. Die Höhe der Einsatzbleche 2 ist dabei so gewählt, dass zwischen dem Boden 18B eines Einsatzbleches 2 und den darunter befindlichen Oberflächen der Packungen P bei Beladetemperatur ein schmaler Luftspalt G vorhanden ist, wie aus Fig. 4 hervorgeht.

## Patentansprüche

1. Quaderförmige Karton/Kunststoff-Verbundpackung zur Verwendung in einer Vorrichtung zum Sterilisieren von quaderförmigen Karton/Kunststoff Verbundpackungen in einem Autoklaven, wobei die aus Produkt und Verpackung bestehenden Packungen einer Wärmebehandlung bei einer bestimmten Temperatur über eine bestimmte Dauer unterzogen wird, wobei das Produkt aus einer Flüssigkeit und stückigen Anteilen besteht und wobei im Inneren des Autoklaven Einrichtungen zum rotatorischen Antrieb der Packungen um eine Drehachse vorgesehen sind, wobei eine Mehrzahl von in parallelen Reihen dicht benachbart angeordneter Packungen (P) auf Tragböden angeordnet sind und wobei eine Mehrzahl von mit Packungen (P) bestückten Tragböden übereinander angeordnet und in ihrer gegenseitigen Lage fixiert sind,
**dadurch gekennzeichnet, dass**
der Karton zur Verminderung des Wassereintritts mittels Leim hydrophobiert ist und dass die eingesetzte Leimmenge zwischen 1 und 4 kg/t trockenen Faserstoffs liegt.

2. Packung nach Anspruch 1,
**dadurch gekennzeichnet, dass**
die Dichte des Rohkartons im Bereich von 680 bis 860 kg/m³ liegt.

3. Packung nach Anspruch 1 oder 2,
**dadurch gekennzeichnet, dass**
der Mahlgrad der für die Packung (P) verwendeten Kartonfasern im Bereich von 15 bis 35 SR liegt.

4. Packung nach einem der Ansprüche 1 bis 3,
**dadurch gekennzeichnet, dass**
ihre Oberfläche hydrophob bedruckt ist.

5. Packung nach einem der Ansprüche 1 bis 4,
**dadurch gekennzeichnet, dass**
ihre Oberfläche durch eine transparente Kunststoffschicht geschützt ist.

6. Packung nach einem der Ansprüche 1 bis 5,
**dadurch gekennzeichnet, dass**
die Füllmenge so gewählt ist, dass im Inneren der Packung (P) eine Gasblase von 2 bis 30 % des Nennvolumens der Packung (P) vorhanden ist.

7. Packung nach einem der Ansprüche 1 bis 6,
**dadurch gekennzeichnet, dass**
die Schnittkanten der Packung (P) zusätzlich verpresst sind.

8. Packung nach einem der Ansprüche 1 bis 7,
**dadurch gekennzeichnet, dass**
zum Abfangen des Sauerstoffanteils im Karton/Kunststoff-Verbundmaterial Scavenger eingesetzt werden.

## Claims

1. Square-shaped cardboard/plastic compound package for use in a device for sterilising square-shaped cardboard/plastic compound packages in an autoclave, wherein the packages consisting of product and packaging are subjected to a heat treatment at a certain temperature over a certain period of time, wherein the product consists of a liquid and solid parts and wherein inside the autoclave means are provided for the rotary drive of the packages around an axis of rotation, wherein a plurality of packages (P) arranged close to one another in parallel rows are arranged on trays and wherein a plurality of trays fitted with packages (P) are arranged above one another and are fixed in their mutual position,
**characterised in that**
the cardboard, in order to reduce the entry of water, is hydrophobised by means of glue and that the amount of glue used is between 1 and 4 kg/t of dry pulp.

2. Package according to Claim 1,
**characterised in that**
the density of the raw cardboard lies in the range of 680 to 860 kg/m3.

3. Package according to Claim 1 or 2,
**characterised in that**
the grinding degree of the cardboard fibres used for the package (P) lies in the range of 15 to 35 SR.

4. Package according to any one of Claims 1 to 3,
**characterised in that**
its surface is printed hydrophobic.

5. Package according to any one of Claims 1 to 4,
**characterised in that**
its surface is protected by a transparent plastic layer.

6. Package according to any one of Claims 1 to 5,
**characterised in that**
the filling quantity is chosen such that inside the package (P) a gas bubble from 2 up to 30% of the nominal volume of the package (P) is present.

7. Package according to any one of Claims 1 to 6,
**characterised in that**
the cut edges of the package (P) are additionally compressed.

8. Package according to any one of Claims 1 to 7,
**characterised in that**
in order to capture the oxygen content in the cardboard/plastic compound material scavengers are used.

## Revendications

1. Paquet composite carton / matière synthétique carré, destiné à être utilisé dans un dispositif de stérilisation de paquets composites carton / matière synthétique carrés, dans un autoclave, sachant que les paquets, qui consistent en produit et emballage, sont soumis à un traitement à chaud à une température déterminée, pendant une durée déterminée, sachant que le produit consiste en une partie liquide et une partie particules et sachant que des installations sont prévues à l'intérieur de l'autoclave pour entraîner les paquets en rotation autour d'un axe de rotation, sachant qu'une pluralité de paquets (P), disposés en rangées parallèles, étroitement voisines, est agencée sur des supports et sachant qu'une pluralité de supports chargés de paquets (P) sont disposés les uns au-dessus des autres et fixés dans leur position réciproque,
**caractérisé en ce que** le carton est hydrophobé au moyen d'une colle pour réduire l'absorption d'eau et que la quantité de colle employée est située entre 1 à 4 kg / t de matériau fibreux sec.

2. Paquet selon la revendication 1,
**caractérisé en ce que** la densité du carton brut est située dans un domaine de 680 à 860 kg/m3.

3. Paquet selon revendication 1 ou 2,
**caractérisé en ce que** le degré de broyage des fibres de carton, employées pour le paquet (P), est situé dans un domaine de 15 à 35 SR.

4. Paquet selon l'une des revendications 1 à 3,
**caractérisé en ce que** sa surface est pourvue d'une impression hydrophobe.

5. Paquet selon l'une des revendications 1 à 4,
**caractérisé en ce que** sa surface est protégée par une couche de matière synthétique transparente.

6. Paquet selon l'une des revendications 1 à 5,
**caractérisé en ce que** le volume de remplissage est choisie de manière à ce qu'une bulle de gaz de 2 à 30 % du volume nominal de l'emballage (P) soit présente à l'intérieur du paquet (P).

7. Paquet selon l'une des revendications 1 à 6,
**caractérisé en ce que** les bords de coupe de l'emballage (P) sont pressées en supplément.

8. Paquet selon l'une des revendications 1 à 7,
**caractérisé en ce que**, pour intercepter la part d'oxygène dans le matériau composite carton / matière synthétique, on utilise un scavenger.
